(19) Europäisches Patentamt
European Patent Office
Office européen des brevets

(11) Numéro de publication: **0 231 138**
**B1**

(12) **FASCICULE DE BREVET EUROPEEN**

(45) Date de publication du fascicule du brevet: **18.04.90**

(51) Int. Cl.[5]: **C 07 D 471/04,** A 61 K 31/435
// (C07D471/04, 235:00, 221:00)

(21) Numéro de dépôt: **87400074.8**

(22) Date de dépôt: **14.01.87**

(54) **Dérivés d'acylaminométhyl-1imidazo 1,2-ar quinoléines, leur préparation et leur application en thérapeutique.**

(30) Priorité: **22.01.86 FR 8600835**

(43) Date de publication de la demande:
**05.08.87 Bulletin 87/32**

(45) Mention de la délivrance du brevet:
**18.04.90 Bulletin 90/16**

(84) Etats contractants désignés:
**AT BE CH DE ES FR GB GR IT LI LU NL SE**

(56) Documents cités:
**EP-A-0 172 096**
**GB-A- 991 589**

(73) Titulaire: **SYNTHELABO**
**58 rue de la Glacière**
**F-75013 Paris (FR)**

(72) Inventeur: **George, Pascal**
**39, rue Henri de Vilmorin**
**F-94400 Vitry S/Seine (FR)**
Inventeur: **de Peretti, Danièle**
**14, rue Ampère**
**F-92160 Antony (FR)**

(74) Mandataire: **Ludwig, Jacques et al**
**SYNTHELABO Service Brevets 58, rue de la Glacière**
**F-75621 Paris Cedex 13 (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 231 138 B1

## Description

La présente invention a pour objet des dérivés d'acylaminométhyl-1 imidazo[1,2-*a*]quinoléines de formule (I), figurant dans le shéma de l'annexe 1, formule dans laquelle A et B représentent chacun un atome d'hydrogène ou ensemble une liaison carbone-carbone,

X représente un atome d'hydrogène ou d'halogène ou un groupe $(C_{1-4})$alkyle, $(C_{1-4})$alkoxy, $(C_{1-4})$alkylthio, méthylsulfonyle, amino, $(C_{1-4})$alkylamino, di-$(C_{1-4})$alkylamino, nitro ou trifluorométhyle,

Y représente un atome d'hydrogène ou d'halogène ou un groupe méthyle en position 6, 7 ou 8,

$R_1$ représente un atome d'hydrogène ou un radical $(C_{1-4})$alkyle, et

$R_2$ représente un radical $(C_{1-6})$alkyle droit ou ramifié.

Les composés préférés de l'invention sont ceux dans lesquels

X est un atome de chlore ou le groupe méthyle ou méthylthio,

Y est un atome d'hydrogène,

$R_1$ est un atome d'hydrogène ou le radical méthyle,

A, B et $R_2$ ont les significations précédemment données.

Les sels d'addition que peuvent former les composés avec des acides pharmacologiquement acceptables font partie de l'invention.

Les composeés de l'invention peuvent être préparés selon le schéma de l'annexe 1.

On soumet d'abord la quinoléine de formule (II) à l'action d'une α-bromo-acétophénone portant le substituant X défini ci-dessus, à chaud, dans un solvant tel que le chlorure de méthylène ou le dichloro-1,2 éthane. On obtient un composé ionique de formule (III) que l'on cyclise à chaud

soit en présence d'acétate d'ammonium, dans un solvant organique tel que l'acide acétique ou propionique, à une température de 90°C de manière à obtenir les composés (IV) dans lesquels A et B sont des atomes d'hydrogène,

soit en présence d'acétate d'ammonium, et de chlorure ferrique, dans un solvant tel que l'acide acétique ou propionique, à une température de 120°C et 140°C, de manière à obtenir les composés (IV) dans lesquels A et B représentent ensemble une liaison carbone-carbone.

On hydroxyméthyle en position 1 le composé (IV) à l'aide d'un excés de formol en milieu acide acétique. On fait réagir l'alcool obtenu (V) avec un nitrile $R_2CN$ en présence d'acide sulfurique concentré et après hydrolyse on obtient le composé (I) dans lequel $R_1$ est H et que l'on alkyle éventuellement à l'aide d'un iodure d'alkyle $R_1I$.

Les exemples suivants illustrent l'invention.

Les microanalyses et les spectres IR et RMN confirment la structure des composés.

### Exemple 1

*N*-méthyl-*N*-[[(méthyl-4 phényl)-2 dihydro-4,5 imidazo[1,2-*a*]quinoléinyl-1]méthyl]butanamide

1.1. Bromure de [(méthyl-4 phényl)-2 oxo-2 éthyl]-1 quinoléinium.

On dissout 110 g (0,516 mol) d'α-bromo-para-méthyl-acétophénone et 61 ml (0,516 mole) de quinoléine dans 500 ml de chlorure de méthylène. On chauffe la solution à la température du reflux pendant 1 heur puis on la dilue avec 300 ml d'éther et la refroidit. On obtient un précipité jaune que l'on filtre et sèche. F = 220—221°C.

1.2. (Méthyl-4 phényl)-2 dihydro-4,5 imidazo[1,2-*a*]quinoléine.

On mélange, dans 50 ml d'acide acétique, 17,1 g (0,05 mole) du sel quaternaire obtenu sous 1.1 et 25 g d'acétate d'ammonium. On chauffe cette suspension à 90°C pendant 3 heures puis la refroidit. On y ajoute 200 ml d'eau et filtre le précipité brun. On reprend ce dernier avec de l'eau et du chlorure de méthyléne et on traite ce mélange biphasique par un excès de NaOH 1N. On décante la phase aqueuse, la sèche au $Na_2SO_4$ et la filtre. On concentre le filtrat sous pression réduite. On obtient, après recristallisation dans du pentane, le composé (IV). F = 91—2°C (décomposition).

1.3. (méthyl-4-phényl)-2 dihydro-4,5 imidazo[1,2-*a*]quinoléine-1-méthanol.

Dans 62 ml d'acide acétique glacial, on dissout 4,62 g (0,0164 mole) du composé obtenu en 1.2., 12,5 ml de formaldéhyde à 37% dans l'eau et on chauffe le mélange pendant 4h30 à 50°C. On concentre le mélange réactionnel sous pression réduite, on reprend a résidu à l'eau et à l'ammoniaque jusqu'à pH≥7. On extrait le composé obtenu avec du chlorure de méthylène. Après séchage et évaporation du solvent on obtient un solide beige. F = 213,5—216°C.

1.4. *N*-[[(méthyl-4 phényl)-2 dihydro-4,5 imidazo[1,2-*a*]quinoléinyl-1]méthyl]butanamide.

Dans une suspension de 3,4 g du composé obtenu sous 1.3. dans 34 ml de butyronitrile que l'on maintient à 50°C, on ajoute goutte à goutte 2,9 ml d'acide sulfurique concentré. On augmente progressivement le chauffage jusqu'à ce qu'une huile décante. On élimine l'excès de butyronitrile, on hydrolyse l'huile, puis la traite avec un excès d'ammoniaque jusqu'à pH basique. On extrait le composé obtenu au chlorure de méthylène puis le purifie par chromatographie sur silice et enfin, le fait recristalliser dans de l'acétate d'éthyle. F = 212—213°C.

1.5. *N*-méthyl-*N*-[[(méthyl-4 phényl)-2-dihydro-4,5 imidazo[1,2-*a*]quinoléinyl-1]méthyl]butanamide.

On met en suspension 2 g (0,0056 mole) de l'amide obtenu sous 1.4. dans un mélange de 40 ml de THF sec et de 4 ml de DMF sec contenant 0,560 g (2 équivalents) de NaH à 50% dans de l'huile et 1,62 g (0,01 mole) d'iodure de méthyle. On maintient ce mélange sous agitation jusqu'à la fin du dégagement gazeux, puis ensuite on le traite par 1 ml de méthanol. On évapore le mélange réactionnel sous pression réduite, reprend le résidu à l'eau, extrait le composé (I) au chlorure de méthylène et le purifie par chromatographie. F = 110—112°C.

Exemple 2

*N*-méthyl-*N*-[[(méthyl-4 phényl)-2 imidazo[1,2-*a*]quinoléinyl-1]méthyl]butanamide

2.1. Bromure de [(méthyl-4 phényl)-2 oxo-2 éthyl]-1 quinoléinium.

On dissout 110 g (0,516 mol) d'α-bromo-para-méthyl-acétophénone et 61 ml (0,516 mole) de quinoléine dans 500 ml de chlorure de méthylène. On chauffe la solution à la température du reflux, pendant 1 heure, puis on la dilue avec 300 ml d'éther et la refroidit. On obtient le sel quaternaire (III) par filtration. F = 220—1°C.

2.2. (méthyl-4 phényl)-2 imidazo[1,2-*a*]quinoléine.

On chauffe à la température du reflux, pendant 24 heures, un mélange de 75 g (0,219 mole) du sel obtenu sous 2.1., 102,5 g d'acétate d'ammonium, 112,5 g de chlorure ferrique et 750 ml d'acide propionique. Après refroidissement, on filtre le précipité, le lave 2 fois avec 100 ml d'acide acétique puis à l'eau jusqu'à ce que le liquide qui s'écoule soit incolore. On met en suspension le précipité dans de l'eau et le traite par un excès d'ammoniaque. On extrait le produit au chlorure de méthylène, décante, sèche le phase organique sur $MgSO_4$, filtre et concentre le filtrat sous pression réduite. On purifie le résidu d'évaporation par chromatographie.

Le produit (IV) fond à 118—120°C.

2.3. (méthyl-4-phényl)-2 imidazo[1,2-*a*]quinoléine-1-méthanol.

On dissout 7 g (0,027 mole) du composé obtenu sous 2.2 dans 90 ml d'acide acétique. On y ajoute 19 ml de formol à 37% dans l'eau et on chauffe la solution à 60°C pendant 3 heures. On évapore l'acide acétique sous pression réduite et traite le résidu par 100 ml d'eau et un excès d'ammoniaque (jusqu'à pH≥7). On filtre le composé, le lave à l'eau et au chlorure de méthylène. Après séchage sous vide, on obtient l'alcool (V). F = 287—8°C.

2.4. [[(méthyl-4 phényl)-2 imidazo[1,2-*a*]quinoléinyl-1]méthyl]butanamide.

On met en suspension 2 g (0,0069 mole) de l'alcool obtenu sous 2.3. dans 20 ml de butyronitrile. On y ajoute, goutte à goutte, lentement, 1,72 ml (3,4 g) d'acide sulfurique concentré à une température ne dépassant pas 10°C. On laisse le mélange revenir à la température ambiante puis le chauffe à 65°C et le laisse refroidir. On élimine l'excès de butyronitrile, traite le solide par 50 g de glace puis un excès de $NH_4OH$ jusqu'à pH basique. On filtre le précipité, le lave à l'eau jusqu'à pH neutre puis à l'acétone et à l'éther et on le sèche. Après recristillisation dans du nitrométhane, on obtient le composé (I) dans lequel $R_1$ = H. F = 238—9°C.

2.5. *N*-methyl-*N*-[[(méthyl-4 phényl)-2 imidazo[1,2-*a*]quinoléinyl-1]méthyl]butanamide.

Dans un mélange de 50 ml de THF et de 5 ml de DMF sec contenant 0,56 g (0,011 mole) de NaH à 50% dans de l'huile, on ajoute un mélange de 2 g (0,0056 mole) du composé obtenu en 2.4. et de 1,59 g (0,011 mole) d'iodure de méthyle dissous dans 10 ml de THF/DMF (90/10). En fin de dégagement gazeux, on maintient l'agitation pendant 1 heure. On traite le mélange réactionnel par 1 ml de méthanol et évapore le solvant. On reprend le résidu par de l'eau et du chlorure de méthylène. On décante la phase organique, le sèche sur $Na_2SO_4$ puis la filtre et l'évapore sous pression réduite. On purifie le résidu par chromatographie sur silice. Après séchage, on obtient le composé (I). F = 147—149°C.

Tableau

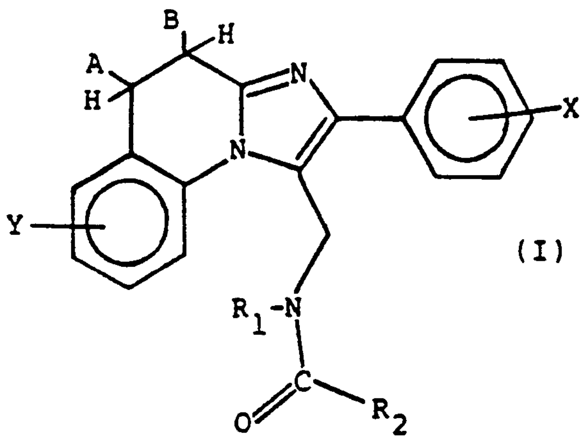

(I)

| Composé | A B | Y | X | $R_1$ | $R_2$ | F(°C) |
|---|---|---|---|---|---|---|
| 1 | H H | H | $4-CH_3$ | H | $CH_3$ | 229-231 |
| 2 | H H | H | $4-CH_3$ | $CH_3$ | $CH_3$ | 144-145 |
| 3 | H H | H | $4-CH_3$ | H | $nC_3H_7$ | 212-213 |
| 4 | H H | H | $4-CH_3$ | $CH_3$ | $nC_3H_7$ | 110-112 |
| 5 | H H | H | $4-CH_3$ | H | $iC_4H_9$ | 204-206 |
| 6 | H H | H | $4-CH_3$ | $CH_3$ | $iC_4H_9$ | 89-91 |
| 7 | H H | H | 4-Cl | H | $nC_3H_7$ | 209-211 |
| 8 | H H | H | 4-Cl | $CH_3$ | $nC_3H_7$ | 76-77 |
| 9 | H H | H | 4-Cl | H | $iC_4H_9$ | 206-207 |
| 10 | H H | H | 4-Cl | $CH_3$ | $iC_4H_9$ | 91-93 |

Tableau (suite)

| Composé | A B | Y | X | $R_1$ | $R_2$ | F(°C) |
|---|---|---|---|---|---|---|
| 11 | H H | H | 4-$SCH_3$ | H | i$C_4H_9$ | 207-209 |
| 12 | H H | H | 4-$SCH_3$ | $CH_3$ | i$C_4H_9$ | 92-93 |
| 13 | —— | H | 4-$CH_3$ | H | $CH_3$ | 275-277 |
| 14 | —— | H | 4-$CH_3$ | $CH_3$ | $CH_3$ | 137-138 |
| 15 | —— | H | 4-$CH_3$ | H | n$C_3H_7$ | 238-239 |
| 16 | —— | H | 4-$CH_3$ | $CH_3$ | n$C_3H_7$ | 147-149 |
| 17 | —— | H | 4-$CH_3$ | H | i$C_4H_9$ | 246-247 |
| 18 | —— | H | 4-$CH_3$ | $CH_3$ | i$C_4H_9$ | 105-106 |
| 19 | —— | H | 4-Cl | H | $CH_3$ | 289-290 |
| 20 | —— | H | 4-Cl | $CH_3$ | $CH_3$ | 179-181 |

Tableau (suite)

| Composé | A B | Y | X | $R_1$ | $R_2$ | F(°C) |
|---|---|---|---|---|---|---|
| 21 | —— | H | 4-Cl | H | $nC_3H_7$ | 270-271 |
| 22 | —— | H | 4-Cl | $CH_3$ | $nC_3H_7$ | 166-167 |
| 23 | —— | H | 4-Cl | H | $iC_4H_9$ | 263-265 |
| 24 | —— | H | 4-Cl | $CH_3$ | $iC_4H_9$ | 138-140 |
| 25 | —— | H | 4-Cl | H | $nC_5H_{11}$ | 218-219 |
| 26 | —— | H | 4-Cl | $CH_3$ | $nC_5H_{11}$ | 143-145 |
| 27 | —— | H | $4-SCH_3$ | H | $nC_3H_7$ | 252-253 |
| 28 | —— | H | $4-SCH_3$ | $CH_3$ | $nC_3H_7$ | 149-150 |
| 29 | —— | H | $4-SCH_3$ | H | $iC_4H_9$ | 241-242 |
| 30 | —— | H | $4-SCH_3$ | $CH_3$ | $iC_4H_9$ | 170-172 |
| 31 | —— | H | $4-NO_2$ | H | $iC_4H_9$ | 261-262 |
| 32 | —— | H | $4-NO_2$ | $CH_3$ | $iC_4H_9$ | 227-228 |

Les composés de l'invention ont fait l'objet d'essais pharmacologiques qui ont mis en évidence leur intérêt comme substances à activités thérapeutiques.

Antagonisme vis-à-vis des convulsions cloniques induites par le Cardiazol® chez la souris.

L'essai est inspiré du protocole décrit par Goodman et al., J. Pharm. Exp. Ther., *108,* 168—176. Les souris reçoivent les produits à tester, ou le solvant seul, par voie intrapéritonéale 30 minutes avant l'injection de 35 mg/kg de Cardiazol® par voie intraveineuse. Les animaux sont ensuite observés pendant une heure et, pour chaque lot, le pourcentage de souris présentant des convulsions cloniques est noté (100% de convulsions cloniques et 10 à 20% de convulsions toniques chez les animaux de contrôle).

Pour chaque dose, on calcule le pourcentage de protection par rapport aux animaux de contrôle, ce qui permet de déterminer graphiquement la $DA_{50}$, dose qui protège 50% des animaux vis-à-vis des effets convulsivants du Cardiazol®. Les $DA_{50}$ des composés de l'invention se situent entre 0,1 et 30 mg/kg pour la voie intrapéritonéale et entre 0,1 et 30 mg/kg pour la voie orale.

Test "d'enfouissement" chez la souris ("Burying test").

Ce test est inspiré de la méthode décrite par Pinel J. P. J., Treit D., Ladak F. et MacLennan A. J. dans Animal learning and behavior, *8,* 447—451, (1980).

La présence de corps étrangers dans l'environnement habituel d'un animal constitue une situation aversive à laquelle l'animal réagit en enfouissant l'objet de l'agression (billes de verre) dans la sciure de sa cage.

Les anxiolytiques ont pour effet de diminuer l'appréhension causée par la présence étrangère: les animaux enfouissent moins. On compte alors le nombre de billes restées non enfouies.

Les produits à étudier sont administrés à des souris mâles de souche CDI (Charles River) 30 minutes (voie intrapéritonéale) ou 60 minutes (voie orale) avent que ces dernières soient placées dans des cages contenant 25 billes de verre. Au bout de 30 minutes on compte le nombre de billes restées non efouies. Un pourcentage est calculé entre les animaux traités et les animaux témoins.

On détermine ainsi la $DA_{50}$, dose active 50%, qui est la dose de composé (en mg/kg) diminuant de moitié le nombre de billes enfouies, encomparaison avec les animaux témoins. Les $DA_{50}$ des composés de l'invention se situent entre 0,3 et 30 mg/kg par voie intrapéritonéale.

Test de conflit de la boisson chez le rat.

Ce test est décrit par Vogel J. R., Beer B. et Clody D. E. dans Psychopharmacologia, *21,* 1—7, (1971).

Des rats mâles Wistar (IFFA Credo) sont utilisés. L'eau de boisson leur est retirée 24 h avant le test. Le jour du test, 30 minutes après traitement par voie intrapéritonéale avec les composés de l'invention, chaque rat est placé dans une cage en matière plastique transparente (24 × 20 × 21 cm) à plancher grillagé électrifiable. De l'eau de boisson est distribuée par l'intermédiaire d'une pipette sortant de 2 cm d'une paroi de la cage et placée à 3 cm au-dessus du plancher de la cage.

Après une exploration de 10 à 90 secondes, l'animal trouve la pipette et commence à boire. Après avoir donné 20 coups de langue (enregistrés par un anxiomètre OMNITECH), le rat reçoit, au niveau de la langue un choc électrique de 0,07 mA (délivré par l'anxiomètre) qui s'arrête lorsque le rat quitte la pipette. Une session de 3 minutes commence après un premier choc, l'animal continuant de recevoir un choc tous les 20 coups de langue jusqu'à ce qu'il s'arrête ou jusqu'à la fin de la session.

Dans ces conditions expérimentales, les animaux de contrôle acceptent, en moyenne, 3 à 6 chocs. Le nombre de chocs obtenus avec les animaux traités est noté, et on compare ce nombre avec celui des animaux témoins par un test de Dunett.

On détermine ainsi la DEM, dose efficace minimale, qui est la première dose augmentant de façon significative le nombre de chocs acceptés par un animal, par rapport aux animaux témoins.

Les DEM se situent entre 3 et 100 mg/kg par voie intrapéritonéale.

Action sur l'électrocorticogramme du rat curarisé ventilé.

L'activité sédative ou hypnotique des composés a été déterminée par l'observation de leur action sur l'électrocorticogramme du rat selon la méthode décrite par H. Depoortere, Rev. E. E. G. Neurophysiol., *10,* 3, 207—214 (1980) et per H. Depoortere et M. Decobert, J. Pharmacol. (Paris), *14,* 2, 195—265 (1983).

Les produits à étudier ont été administrés par voie intrapéritonéale aux doses croissantes de 1 à 30 mg/kg.

Ils induisent des tracés de sommeil à partir de doses allant de 3 à 100 mg/kg.

Test de Koster.

L'activité analgésique a été montrée dans le test de Koster et al ("writhing test" à l'acide acétique chez la souris), Fed. Proc., *18,* 412, 1959.

On administre par voie orale, aux souris à jeun, le composé à tester en solution dans du Tween 80 à 1%, à raison de 0,2 ml par 20 g de poids corporel; au bout de 30 mn on administre l'acide acétique (en solution à 0,6% dans un mélange carboxyméthyl-cellulose et tween 80, à raison de 10 ml par kg de poids corporel) par voie intrapéritonéale. On note le nombre total de contorsions pendant 15 mn. On détermine le pourcentage de protection par rapport à un lot témoin et on calcule la DA 50 par voie graphique (dose qui protège 50% des animaux).

La DA 50 des composés de l'invention va de 5 à 50 mg/kg p.o.

Test anti-ulcère de stress.

La technique utilisée est celle de Senay et Levine, Proc. Soc. Exp. Biol. 1967, *124*, 1221—1223, Peptic Ulcers, sur des rats Wistar femelles pesant 180—210 g, tenus à jeun depuis 20 heures, répartis en blocs randomisés.

Les animaux sont mis en contention dans des boîtes cylindriques de 20 cm × 50 cm et placés dans une chambre froide dont la température est maintenue entre 2 et 4°C.

Les composés à étudier sont administrés par voie orale à raison de 10, 30 et 100 mg/kg immédiatement avant la mise en contention, les rats témoins recevant seulement le placébo. 2 heures plus tard, les animaux sont sacrificés par inhalation de chloroforme.

Les estomacs sont prélevés et le degré d'ulcération est noté.

Les composés de l'invention diminuent significativement les ulcères de stress.

Les résultats de ces différents tests montrent que les composés de l'invention possèdent des propriétés anxiolytiques, inductrices de sommeil, hypnotiques, anticonvulsivantes, analgésiques et antiulcères; les composés de l'invention sont utiles pour le traitement des états d'anxiété, des troubles du sommeil et autres affections neurologiques et psychiatriques, pour le traitement des troubles de la vigilance, en particulier pour lutter contre les troubles du comportement imputables à des dommages vasculaires cérébraux et à la sclérose cérébrale en gériatrie, pour le traitement des absences dues à des traumatismes crâniens et pour le traitement des encéphalopathies métaboliques ainsi que pour le traitement des algies, de la douleur et des ulcères.

Les composés de l'invention peuvent être présentés sous forme appropriée pour l'administration par voie orale ou parentérale, par exemple sous forme de comprimés, de dragées, de gélules, de solutions buvables ou injectables etc, en association avec tout excipient approprié. La posologie quotidienne peut aller de 1 à 100 mg.

Annexe I

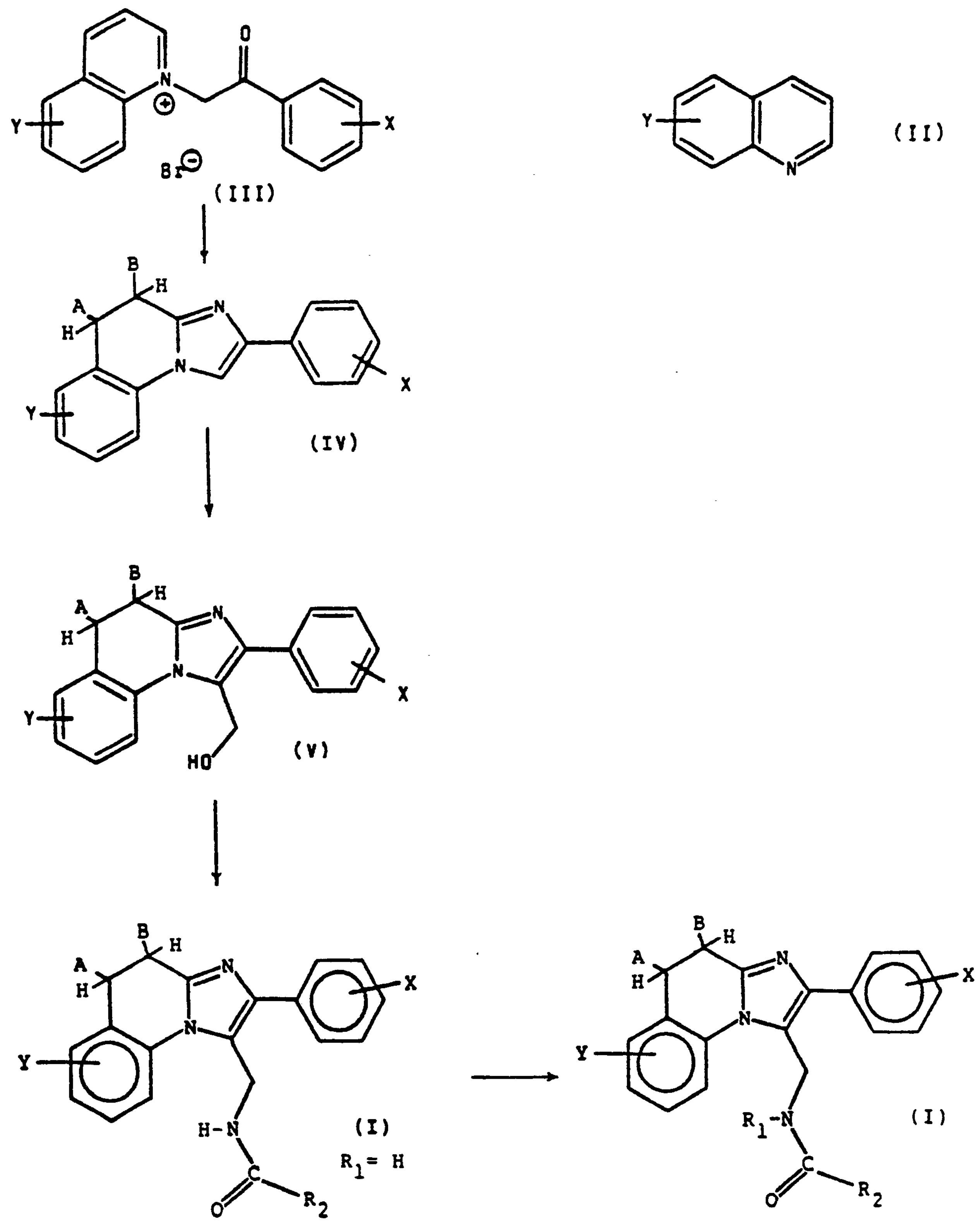

(III)

(II)

(IV)

(V)

(I)

$R_1$= H

(I)

**Revendications pour les États contractants: BE CH DE FR GB IT LI LU NL SE**

1. Dérivés d'acylaminométhyl-1 imidazo[1,2-*a*]quinoléines, répondant à la formule (I)

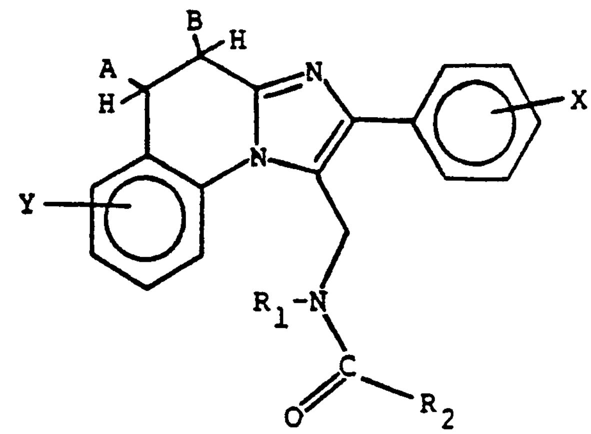

(I)

dans laquelle A et B resprésentent chacun un atome d'hydrogène ou ensemble un liaison carbone-carbone,

X représente un atome d'hydrogène ou d'halogène ou un groupe $(C_{1-4})$alkyle, $(C_{1-4})$alcoxy, $(C_{1-4})$alkylthio, méthylsulfonyle, amino, $(C_{1-4})$alkylamino, di-$(C_{1-4})$alkylamino, nitro ou trifluorométhyle,

Y représente un atome d'hydrogène ou d'halogène ou un groupe méthyle en position 6, 7 ou 8,

$R_1$ représente un atome d'hydrogène ou un radical $C_{1-4}$)alkyle, et

$R_2$ représente un radical $(C_{1-6})$alkyle droit ou ramifié, ainsi que leurs sels d'addition aux acides pharmaceutiquement acceptables.

2. Dérivés selon la revendication 1, caractérisés par le fait que X est un atome de chlore ou le groupe méthyle ou méthylthio, Y est un atome d'hydrogène, $R_1$ est un atome d'hydrogène ou le radical méthyle, et A, B, et $R_2$ ont les significations précédemment données dans la revendication 1.

3. Procédé de préparation des composés de la revendication 1, procédé caractérisé par le fait que l'on soumet d'abord la quinoléine de formule (II)

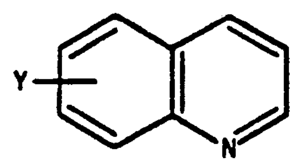

(II)

à l'action d'une α-bromo-acétophénone portant le substituant X défini ci-dessus, à chaud, dans un solvant tel que le chlorure de méthylène ou le dichloro-1,2 éthane; on obtient un composé ionique de formule (III)

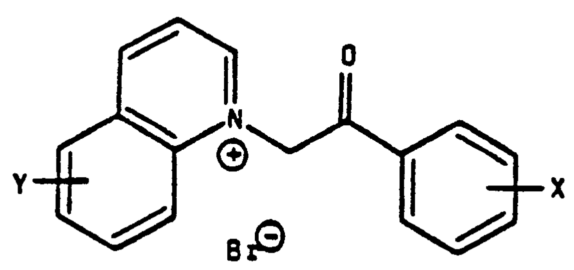

(III)

que l'on cyclise à chaud

soit en présence d'acétate d'ammonium, dans un solvant organique tel que l'acide acétique ou propionique, à une température de 90°C de manière à obtenir les composés (IV)

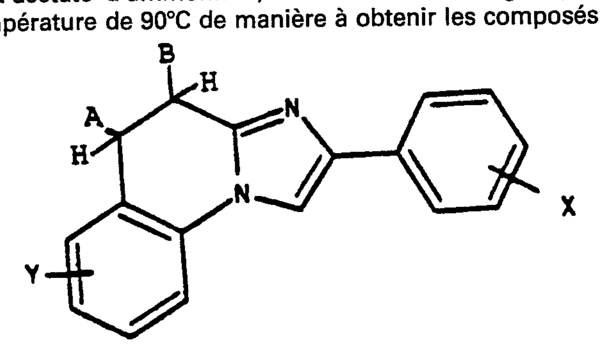

(IV)

dans lesquels A et B sont des atomes d'hydrogène,

soit en présence d'acétate d'ammonium, et de chlorure ferrique, dans un solvant tel que l'acide acétique ou propionique, à une température de 120 à 140°C, de manière à obtenir les composés (IV) dans lesquels A et B représentent ensemble une liaison carbone-carbone; on hydroxyméthyle en position 1 le composé (IV) à l'aide d'un excès de formol en milieu acide acétique; fait réagir l'alcool obtenu (V)

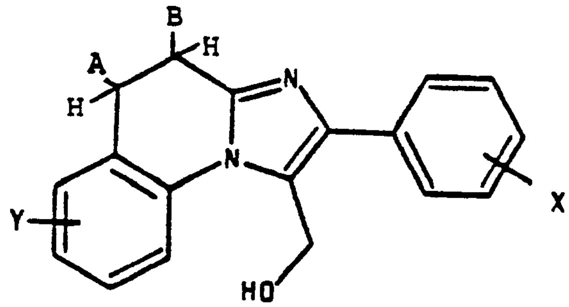

(V)

avec un nitrile $R_2CN$ en présence d'acide sulfurique concentré et, après hydrolyse, on obtient le composé (I) dans lequel $R_1$ est H et que l'on alkyle éventuellement à l'aide d'un iodure d'alkyle $R_1I$.

4. Medicament, caractérisé en ce qu'il est constitué par un composé selon l'une des revendications 1 et 2.

5. Composition pharmaceutique, caractérisée en ce qu'elle contient un composé selon l'une des revendications 1 et 2 en association avec un excipient approprié.

**Revendications pour les États contractants: AT ES GR**

1. Procédé de préparation de dérivés d'acylaminométhyl-1 imidazo[1,2-*a*]quinoléines, répondant à la formule (I)

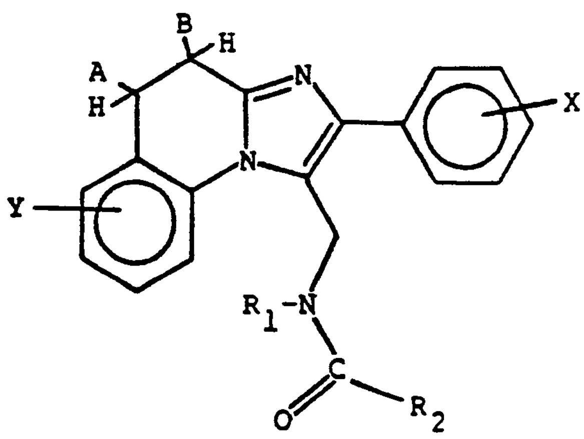

(I)

dans laquelle A et B resprésentent chacun un atome d'hydrogène ou ensemble un liaison carbone-carbone,

X représente un atome d'hydrogène ou d'halogène ou un groupe $(C_{1-4})$alkyle, $(C_{1-4})$alcoxy, $(C_{1-4})$alkylthio, méthylsulfonyle, amino, $(C_{1-4})$alkylamino, di-$(C_{1-4})$alkylamino, nitro ou trifluorométhyle,

Y représente un atome d'hydrogène ou d'halogène ou un groupe méthyle en position 6, 7 ou 8,

$R_1$ représente un atome d'hydrogène ou un radical $C_{1-4})$alkyle, et

$R_2$ représente un radical $(C_{1-6})$alkyle droit ou ramifié, procédé caractérisé par le fait que l'on soumet d'abord la quinoléine de formule (II)

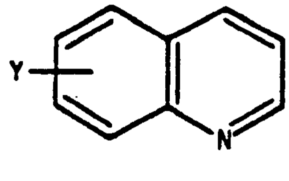

(II)

à l'action d'une α-bromo-acétophénone portant le substituant X défini ci-dessus, à chaud, dans un solvant tel que le chlorure de méthylène ou le dichloro-1,2 éthane; on obtient un composé ionique de formule (III)

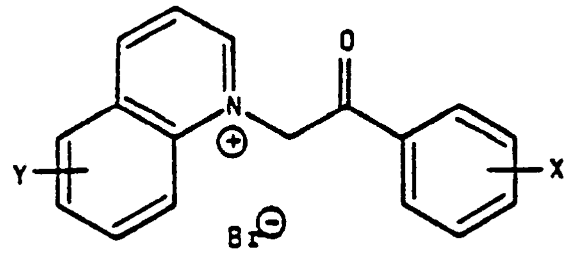

(III)

que l'on cyclise à chaud

soit en présence d'acétate d'ammonium, dans un solvant organique tel que l'acide acétique ou propionique, à une température de 90°C de manière à obtenir les composés (IV)

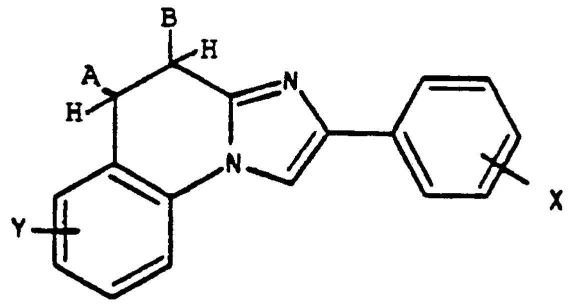

(IV)

dans lesquels A et B sont des atomes d'hydrogène,

soit en présence d'acétate d'ammonium, et de chlorure ferrique, dans un solvant tel que l'acide acétique ou propionique, à une température de 120 à 140°C, de manière à obtenir les composés (IV) dans lesquels A et B représentent ensemble une liaison carbone-carbone; on hydroxyméthyle en position 1 le composé (IV) à l'aide d'un excès de formol en milieu acide acétique; fait réagir l'alcool obtenu (V)

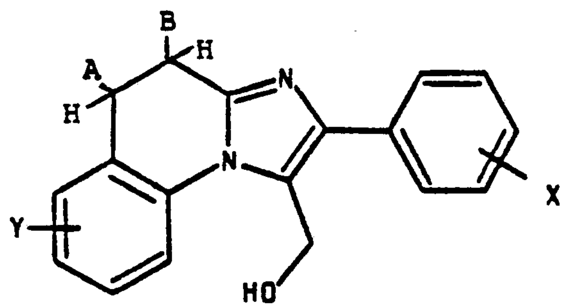

(V)

avec un nitrile $R_2CN$ en présence d'acide sulfurique concentré et, après hydrolyse, on obtient le composé (I) dans lequel $R_1$ est H et que l'on alkyle éventuellement à l'aide d'un iodure d'alkyle $R_1I$.

2. Procédé selon la revendication 1, caractérisés par le fait que X est un atome de chlore ou le groupe méthyle ou méthylthio, Y est un atome d'hydrogène, $R_1$ est un atome d'hydrogène ou le radical méthyle, et A, B, et $R_2$ ont les significations précédemment données dans la revendication 1.

**Patentansprüche für die Vertragsstaaten: BE CH DE FR GB IT LI LU NL SE**

1. Derivate von 1-Acylaminomethyl-imidazo[1,2-a]chinolinen gemäß der Formel (I)

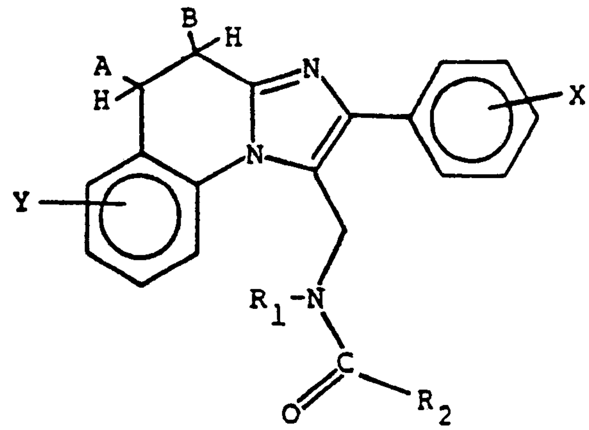

(I)

wobei A und B jeweils ein Wasserstoffatom oder gemeinsam eine Kohlenstoff-Kohlenstoffbindung darstellen, X ein Wasserstoffatom oder ein Halogenatom oder eine der Gruppen $(C_{1-4})$-Alkyl, $C_{1-4})$-Alkoxy, $(C_{1-4})$-Alkylthio, Methylsulfonyl, Amino, $(C_{1-4})$-Alkylamino, Di-$(C_{1-4})$-alkylamino, Nitro oder Trifluoromethyl darstellt,

Y ein Wasserstoffatom oder ein Halogenatom oder eine Methylgruppe in Stellung 6, 7 oder 8 darstellt,

$R_1$ ein Wasserstoffatom oder ein $(C_{1-4})$-Alkylgruppe darstellt und

$R_2$ eine gerade oder verzweigte $(C_{1-6})$-Alkylgruppe darstellt,

sowie ihre Additionssalze mit pharmazeutisch verwendbaren Säuren.

2. Derivate nach Anspruch 1, dadurch gekennzeichnet, daß X ein Chloratom oder die Gruppe Methyl oder Methylthio ist, Y ein Wasserstoffatom ist, $R_1$ ein Wasserstoffatom oder die Methylgruppe ist, und A, B und $R_2$ die in Anspruch angegebenen Bedeutungen haben.

3. Verfahren zur Herstellung der Verbindungen nach Anspruch 1, dadurch gekennzeichnet, daß zunächst ein Chinolin der Formel (II)

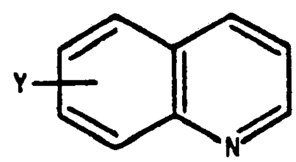

(II)

der Einwirkung eines α-Bromacetophenons, das den oben definierten Substituenten X trägt, in der Hitze in einem Lösungsmittel wie Methylenchlorid oder 1,2-Dichloräthan unterworfen wird;

eine ionische Verbindung der Formel (III) erhalten wird

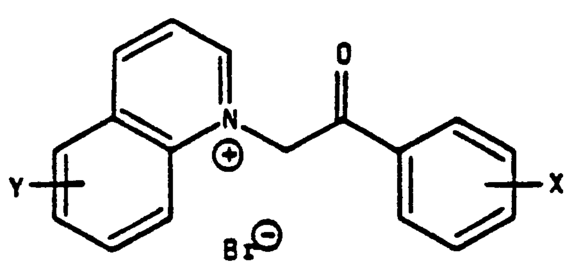

(III)

die in der Hitze zyklisiert wird,

entweder ion Gegenwart von Ammoniumacetat in einem organischen Lösungsmittel wie Essigsäure oder Propionsäure bei einer Temperatur von 90°C, um die Verbindungen (IV) zu erhalten

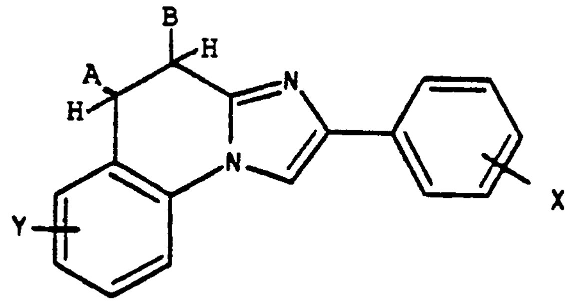

(IV)

wobei A und B Wasserstoffatome darstellen,

oder in Gegenwart von Ammoniumacetat und Eisen(III)chlorid in einem Lösungsmittel wie Essigsäure oder Propionsäure bei einer Temperatur von 120 bis 140°C, um die Verbindungen (IV) zu erhalten, wobei A und B gemeinsam eine Kohlenstoff-Kohlenstoffbindung darstellen;

die Verbindung (IV) in Stellung 1 hydroxymethyliert wird mit Hilfe eines Überschusses an Formaldehyd in essigsaurem Milieu;

der erhlatene Alkohol (V)

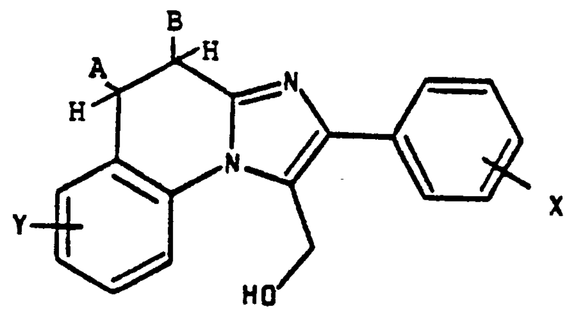

(V)

mit einem Nitril $R_2CN$ in Anwesenheit von konzentrierter Schwefelsäure zur Reaktion gebracht und nach Hydrolyse die Verbindung (I) erhalten wird, wobei $R_1$ Wasserstoff ist, und die gegebenenfalls mit Hilfe eines Alkyljodids $R_1J$ alkyliert wird.

4. Arzneimittel, dadurch gekennzeichnet, daß es aus einer Verbindung nach einem der Ansprüche 1 und 2 besteht.

5. Pharmazeutische Zubereitung, dadurchgekennzeichent, daß sie eine Verbindung nach einem der Ansprüche 1 und 2 gemeinsam mit einem geeigneten Exzipiens enthält.

**Patentansprüche für die Vertragsstaaten: AT ES GR**

1. Verfahren zur Herstellung von Derivaten von 1-Acylaminomethyl-imidazo[1,2-a]chinolinen gemäß der Formel (I)

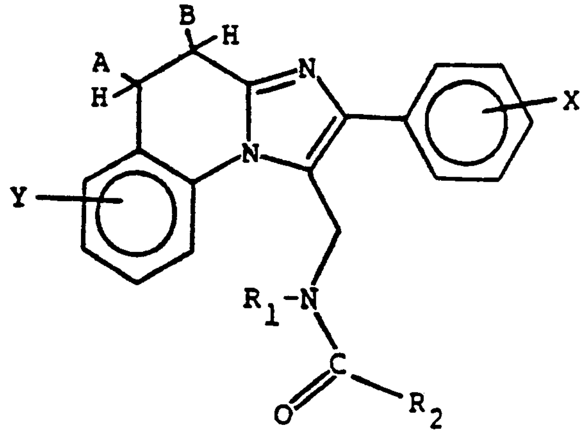

(I)

wobei A und B jeweils ein Wasserstoffatom oder gemeinsam eine Kohlenstoff-Kohlenstoffbindung darstellen, X ein Wasserstoffatom oder ein Halogenatom oder eine der Gruppen $(C_{1-4})$-Alkyl, $C_{1-4})$-Alkoxy, $(C_{1-4})$-Alkylthio, Methylsulfonyl, Amino, $(C_{1-4})$-Alkylamino, Di-$(C_{1-4})$-alkylamino, Nitro oder Trifluoromethyl darstellt,

Y ein Wasserstoffatom oder ein Halogenatom oder eine Methylgruppe in Stellung 6, 7 oder 8 darstellt,

$R_1$ ein Wasserstoffatom oder ein $(C_{1-4})$-Alkylgruppe darstellt und

$R_2$ eine gerade oder verzweigte $(C_{1-6})$-Alkylgruppe darstellt,

dadurch gekennzeichnet, daß zunächst ein Chinolin der Formel (II)

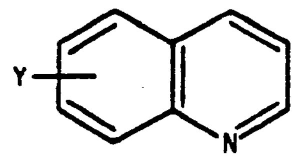

(II)

der Einwirkung eines α-Bromacetophenons, das den oben definierten Substituenten X trägt, in der Hitze in einem Lösungsmittel wie Methylenchlorid oder 1,2-Dichloräthan unterworfen wird;

eine ionische Verbindung der Formel (III) erhalten wird

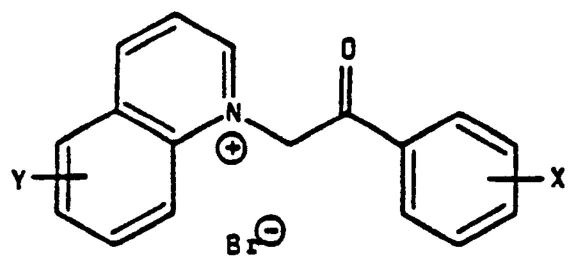

(III)

die in der Hitze zyklisiert wird,

entweder ion Gegenwart von Ammoniumacetat in einem organischen Lösungsmittel wie Essigsäure oder Propionsäure bei einer Temperatur von 90°C, um die Verbindungen (IV) zu erhalten

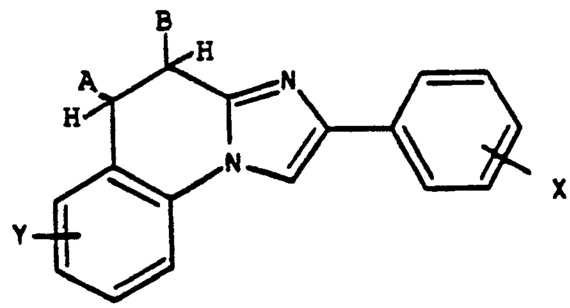

(IV)

wobei A und B Wasserstoffatome darstellen,

oder in Gegenwart von Ammoniumacetat und Eisen(III)chlorid in einem Lösungsmittel wie Essigsäure oder Propionsäure bei einer Temperatur von 120 bis 140°C, um die Verbindungen (IV) zu erhalten, wobei A und B gemeinsam eine Kohlenstoff-Kohlenstoffbindung darstellen;

die Verbindung (IV) in Stellung 1 hydroxymethyliert wird mit Hilfe eines Überschusses an Formaldehyd in essigsaurem Milieu;

der erhaltene Alkohol (V)

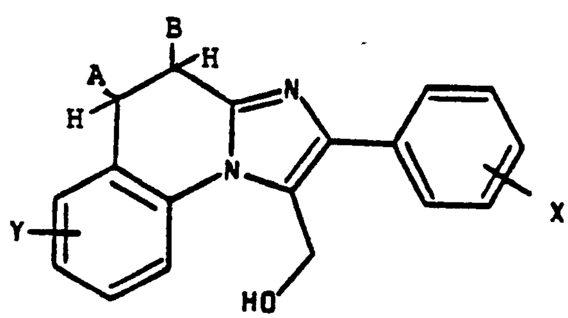

(V)

mit einem Nitril $R_2CN$ in Anwesenheit von konzentrierter Schwefelsäure zur Reaktion gebracht und nach Hydrolyse die Verbindung (I) erhalten wird, wobei $R_1$ Wasserstoff ist, und die gegebenenfalls mit Hilfe eines Alkyljodids $R_1J$ alkyliert wird.

2. Verfahren nach Anspruch 1, dadurch gekennzeichnet, daß X ein Chloratom oder die Gruppe Methyl oder Methylthio ist, Y ein Wasserstoffatom ist, $R_1$ ein Wasserstoffatom oder die Methylgruppe ist, und A, B und $R_2$ die in Anspruch angegebenen Bedeutungen haben.

**Claims for the Contracting States: BE CH DE FR GB IT LI LU NL SE**

1. 1-Acylaminomethyl-imidazo[1,2-*a*]quinoline derivatives, corresponding to the formula (I)

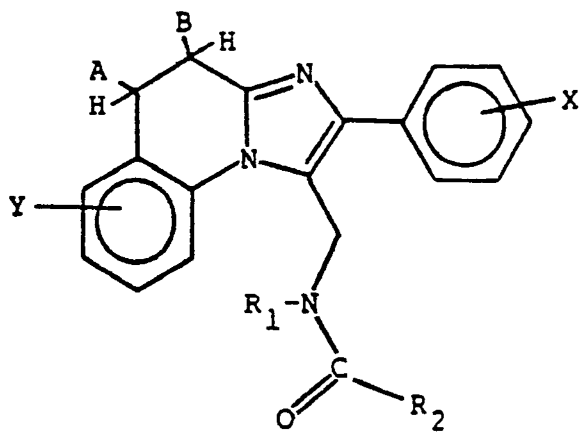

(I)

in which A and B each donate a hydrogen atom or together denote a carbon-carbon bond,

X denotes a hydrogen or halogen atom or a $(C_{1-4})$alkyl, $(C_{1-4})$alkoxy, $(C_{1-4})$alkylthio, methylsulfonyl, amino, $(C_{1-4})$alkylamino, di$(C_{1-4})$alkylamino, nitro or trifluoromethyl group,

Y denotes a hydrogen or halogen atom or a methyl group at position 6, 7 or 8,

$R_1$ denotes a hydrogen atom or a $(C_{1-4})$alkyl radical, and

$R_2$ denotes a linear or branched $(C_{1-6})$alkyl radical, as well as their addition salts with pharmaceutically acceptable acids.

2. Derivatives according to Claim 1, characterized in that X is a chlorine atom or a methyl or methylthio group, Y is a hydrogen atom, $R_1$ is a hydrogen atom or a methyl radical, and A, B and $R_2$ have the meanings given above in Claim 1.

3. Process for preparing the compounds of Claim 1, which process is characterized in that the quinoline of formula (II)

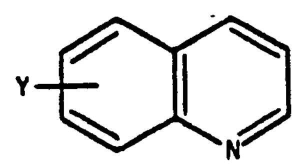

(II)

is first subjected to the action of an α-bromoacetophenone bearing the substituent X defined above, in the heated state, in a solvent such as methylene chloride or 1,2-dichloroethane;

an ionic compound of the formula (III)

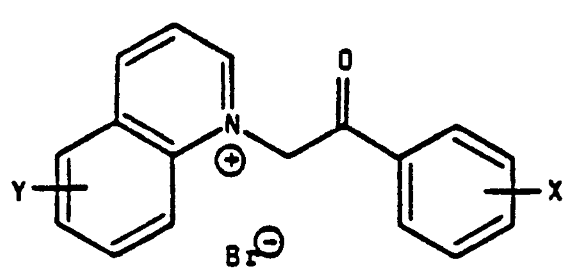

(III)

is obtained, which is cyclized ion the heated state

either in the presence of ammonium acetate, in an organic solvent such as acetic or propionic acid, at a temperature of 90°C, so as to obtain the compounds (IV)

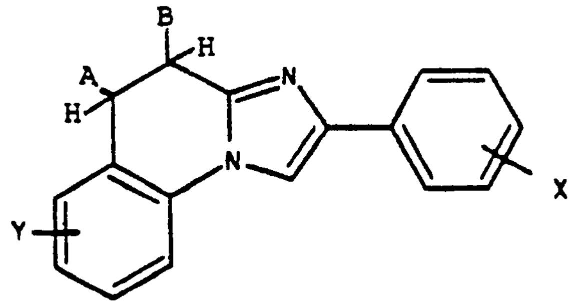

(IV)

in which A and B are hydrogen atoms,

or in the presence of ammonium acetate and ferric chloride, in a solvent such as acetic or propionic acid, at a temperature of 120 to 140°C, so as to obtain the compounds (IV) in which A and B together denote a carbon-carbon bond; the compound (IV) is hydroxymethylated at position 1 using an excess of formaldehyde in acetic acid medium; the alcohol obtained (V)

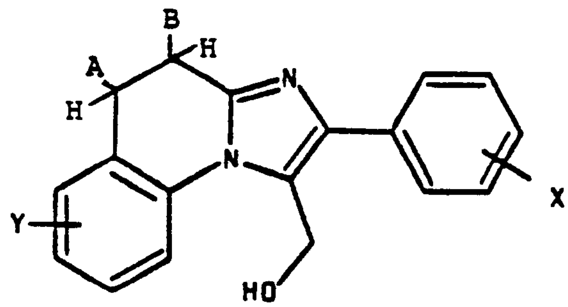

(V)

is reacted with nitrile $R_2CN$ in the presence of concentrated sulphuric acid and, after hydrolysis, compound (I) is obtained in which $R_1$ is H, which compound is optionally alkylated using an alkyl iodide $R_1I$.

4. Drug, characterized in that it consists of a compound according to one of Claims 1 and 2.

5. Pharmaceutical composition, characterized in that it contains a compound according to one of Claims 1 and 2 in combination with a suitable excipient.

**Claims for the Contracting States: AT ES GR**

1. Process for preparing 1-acylaminomethyl-imidazo[1,2-*a*]quinoline derivatives, corresponding to the formula (I)

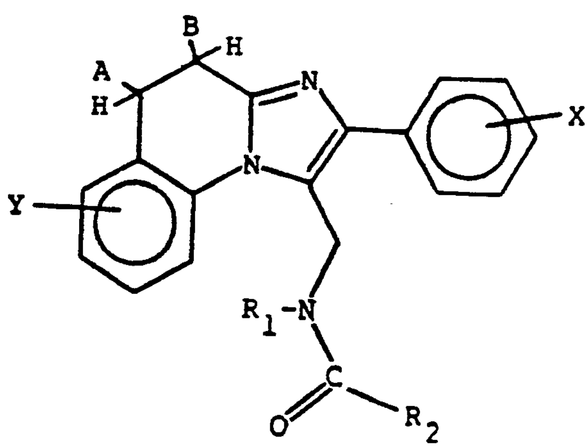

(I)

in which A and B each donate a hydrogen atom or together denote a carbon-carbon bond,

X denotes a hydrogen or halogen atom or a $(C_{1-4})$alkyl, $(C_{1-4})$alkoxy, $(C_{1-4})$alkylthio, methylsulfonyl, amino, $(C_{1-4})$alkylamino, di$(C_{1-4})$alkylamino, nitro or trifluoromethyl group,

Y denotes a hydrogen or halogen atom or a methyl group at position 6, 7 or 8,

$R_1$ denotes a hydrogen atom or a $(C_{1-4})$alkyl radical, and

$R_2$ denotes a linear or branched $(C_{1-6})$alkyl radical, which process is characterized in that the quinoline of formula (II)

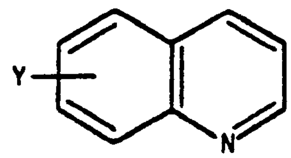

(II)

is first subjected to the action of an α-bromoacetophenone bearing the substituent X defined above, in the heated state, in a solvent such as methylene chloride or 1,2-dichloroethane;

an ionic compound of the formula (III)

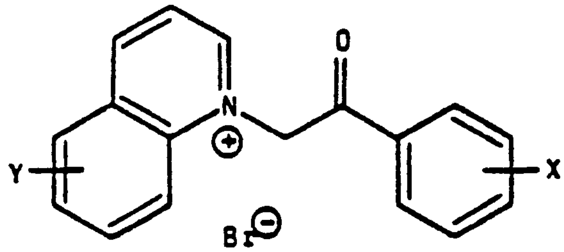

(III)

is obtained, which is cyclized ion the heated state

either in the presence of ammonium acetate, in an organic solvent such as acetic or propionic acid, at a temperature of 90°C, so as to obtain the compounds (IV)

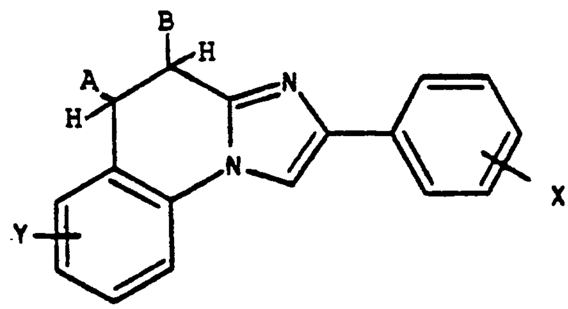

(IV)

in which A and B are hydrogen atoms,

or in the predsence of ammonium acetate and ferric chloride, in a solvent such as acetic or propionic acid, at a temperature of 120 to 140°C, so as to obtain the compounds (IV) in which A and B together denote a carbon-carbon bond; the compound (IV) is hydroxymethylated at position 1 using an excess of formaldehyde in acetic acid medium; the alcohol obtained (V)

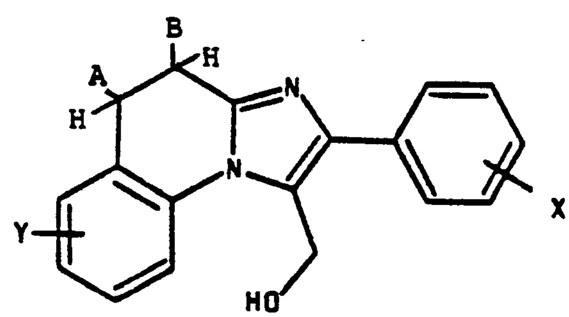

(V)

is reacted with nitrile $R_2CN$ in the presence of concentrated sulphuric acid and, after hydrolysis, compound (I) is obtained in which $R_1$ is H, which compound is optionally alkylated using an alkyl iodide $R_1I$.

2. Process according to Claim 1, characterized in that X is a chlorine atom or a methyl or methylthio group, Y is a hydrogen atom, $R_1$ is a hydrogen atom or a methyl radical, and A, B and $R_2$ have the meanings given above in Claim 1.